# EUROPÄISCHE PATENTANMELDUNG

(11) **EP 3 875 063 A1**
(43) Veröffentlichungstag der Anmeldung: **08.09.2021**
(21) Anmeldenummer: 20160432.9
(22) Anmeldetag: 02.03.2020
(51) Int. Cl.: A61F 4/00, G06F 1/16, H04M 1/05

(54) **EINGABEEINRICHTUNG ZUR VERWENDUNG MIT EINEM COMPUTER**

(71) Anmelder: Majchrzyk-Horacek, Matthias, 55595 Münchwaid (DE); Majchrzyk, Maximilian, 55595 Münchwald (DE)
(72) Erfinder: Majchrzyk-Horacek, Matthias, 55595 Münchwaid (DE); Majchrzyk, Maximilian, 55595 Münchwald (DE)
(74) Vertreter: Franke, Markus

(57) **Zusammenfassung**

Die vorliegende Erfindung betrifft eine Eingabeeinrichtung (1) zur Verwendung mit einem Computer, insbesondere zur Verwendung mit einem Personal Computer (PC). Die Eingabeeinrichtung (1) weist ein Mikrofon (3) und eine Signalverarbeitungseinrichtung (4) auf, wobei die Signalverarbeitungseinrichtung (4) mit dem Mikrofon (3) gekoppelt ist. Die Signalverarbeitungseinrichtung (4) ist dazu eingerichtet, Ausgabesignale des Mikrofons (3) in Tastaturbefehle und/oder in Computermausbefehle umzuwandeln, wobei die Eingabeeinrichtung (1) eine Signalübertragungseinrichtung zum Übertragen der Tastaturbefehle und/oder Computermausbefehle an den Computer aufweist.

## Beschreibung

Die vorliegende Erfindung betrifft eine Eingabeeinrichtung zur Verwendung mit einem Computer, insbesondere zur Verwendung mit einem Personal Computer (PC). Typischerweise dienen als Eingabeeinrichtung für einen Computer, insbesondere einen PC, eine Tastatur und/oder eine Computermaus. Typischerweise sind auch Computerspiele darauf ausgelegt, dass eine Tastatur und/oder eine Computermaus als Eingabeeinrichtungen dienen bzw. das Computerspiel mit einer Tastatur und/oder einer Computermaus steuerbar ist. Die erfindungsgemäße Eingabeeinrichtung dient insbesondere dazu, eine Tastatur und/oder eine Computermaus als Eingabeeinrichtung für den Computer zu ersetzen und/oder zu ergänzen. Die Eingabeeinrichtung dient insbesondere dazu, Menschen mit körperlichen Behinderungen, insbesondere Menschen mit einer Lähmung von Gliedmaßen, die Möglichkeit zu geben, einen Computer, insbesondere einen PC, zu bedienen.

Um Menschen mit einer körperlichen Behinderung die Bedienung eines Computers zu ermöglichen, sind aus dem Stand der Technik unterschiedliche Möglichkeiten bekannt. Aus der WO 2009/078776 A1 ist ein Gerät zum Übertragen von Tatstaturbefehlen von einem Benutzer an einen elektronischen Apparat oder eine elektronische Maschine bekannt, wobei das Gerät eine Vielzahl von Signalsensoren aufweist, die dazu konfiguriert sind, auf die Anwesenheit oder den Druck der Zunge des Benutzers mit Aussenden eines elektrischen Signals oder Impulses zu reagieren, wobei das Gerät ferner eine Signaleinheit aufweist, die dazu konfiguriert ist, die Signale zu registrieren und in korrespondierende Tastaturbefehle umzuwandeln.

Ferner ist aus der EP 1 297 801 A1 eine Steuereinheit bekannt, die insbesondere als Maus- und/oder Tastaturersatz dient, wobei diese Steuereinheit ein mundbetätigbares Stellteil aufweist, das relativ zu einem starren Gehäuse kipp- bzw. verschwenkbar in diesem Gehäuse gelagert ist.

Aufgabe der vorliegenden Erfindung ist es, eine komfortabel zu bedienende Eingabeeinrichtung zur Verwendung mit einem Computer zu schaffen, die ohne Einsatz von Gliedmaßen zu bedienen ist, um insbesondere Menschen mit körperlicher Behinderung die Möglichkeit zu geben, einen Computer zu bedienen. Ferner ist es Aufgabe der Erfindung, die Eingabeeinrichtung derart zu gestalten, dass diese Eingabeeinrichtung dazu eingerichtet ist, unabhängig von dem Endgerät bzw. dem Computer, verwendbar zu sein, sodass es nicht notwendig ist, spezielle Hardware und/oder Software in bzw. auf das Endgerät, das mit der Eingabeeinrichtung bedient werden soll, zu integrieren bzw. zu installieren.

Die erfindungsgemäße Eingabeeinrichtung dient der Verwendung mit einem Computer, insbesondere der Verwendung mit einem PC, wobei die Eingabeeinrichtung ein Mikrofon und eine Signalverarbeitungseinrichtung aufweist, wobei die Signalverarbeitungseinrichtung mit dem Mikrofon gekoppelt ist. Die Signalverarbeitungseinrichtung ist dazu eingerichtet, Ausgabesignale des Mikrofons in Tastaturbefehle und/oder in Computermausbefehle umzuwandeln. Die Eingabeeinrichtung weist ferner eine Signalübertragungseinrichtung zum Übertragen der Tastaturbefehle und/oder Computermausbefehle an den Computer auf.

Die erfindungsgemäße Eingabeeinrichtung ermöglicht es dem Verwender der Eingabeeinrichtung, mittels Geräuschen, insbesondere mittels Sprache, den Computer zu bedienen, wobei es aufgrund der Signalverarbeitungseinrichtung, die dazu eingerichtet ist, Ausgabesignale des Mikrofons in Tastaturbefehle und/oder in Computermausbefehle umzuwandeln, nicht notwendig ist, spezielle Hardware in den Computer zu integrieren und/oder spezielle Software auf dem Computer zu installieren. Der Vorteil einer derartigen Gestaltung der Eingabeeinrichtung besteht darin, dass die Eingabeeinrichtung von dem verwendeten Endgerät bzw. Computer quasi als Tastatur und/oder Computermaus erkannt wird, sodass die erfindungsgemäße Eingabeeinrichtung unkompliziert mit dem jeweiligen Endgerät verwendet werden kann, da die Installation einer speziellen Software und/oder die Integration einer speziellen Hardware entfällt bzw. für die Verwendung der Eingabeeinrichtung nicht notwendig ist. Dies ist insbesondere dann von Vorteil, wenn aus Sicherheitsgründen eine Installation von Software bzw. Gerätetreibern auf dem zu verwendenden Computer nicht möglich ist oder mit einem erhöhten Aufwand verbunden ist.

In einer besonders bevorzugten Ausführungsform der Eingabeeinrichtung weist die Eingabeeinrichtung eine Tragevorrichtung auf, wobei das Mikrofon, vorzugsweise mittels eines verstellbaren Arms, in der Tragevorrichtung gelagert ist, wobei die Tragevorrichtung derart ausgebildet ist, dass diese von einem Verwender im Kopfbereich getragen werden kann. Bei einer derartigen Ausführungsform der Eingabeeinrichtung ist die Eingabeeinrichtung besonders komfortabel zu verwenden, da bei einer derartigen Gestaltung der Eingabeeinrichtung die Positionierung des Mikrofons bezüglich des Mundes des Verwenders quasi unabhängig von Bewegungen des Kopfes und/oder Bewegungen des Körpers des Verwenders ist, sodass sich der Verwender relativ frei bewegen kann, ohne dass dies einen negativen Einfluss auf die Umwandlung vom im Bereich des Mundes gebildeten Lauten, insbesondere Sprache, in Ausgabesignale des Mikrofons hat.

Beispielsweise kann die Tragevorrichtung einen Abschnitt aufweisen, der am Ohr einhängbar ist.

Die Tragevorrichtung kann insbesondere eine bügelförmige Struktur umfassen, wobei es durchaus denkbar ist, dass diese bügelförmige Struktur längenveränderbare Elemente umfasst und/oder in ihrer Krümmung veränderbare Elemente umfasst, um den Tragebügel optimal an die Kopfform des Verwenders anzupassen. Vorzugsweise ist die bügelförmige Struktur dazu ausgebildet, um über dem Kopf getragen zu werden.

In einer besonders bevorzugten Ausführungsform weist die Eingabeeinrichtung zumindest einen Lautsprecher, vorzugsweise einen Kopfhörer, auf. Der Kopfhörer ist vorzugsweise als Muschelkopfhörer ausgebildet.

Der zumindest eine Lautsprecher bzw. der Kopfhörer ist vorzugsweise in der Tragevorrichtung gelagert.

In einer besonders bevorzugten Ausführungsform der Eingabeeinrichtung ist diese als Headset ausgebildet.

In einer vorteilhaften Weiterbildung der Eingabeeinrichtung weist die Eingabeeinrichtung zumindest einen Beschleunigungssensor, vorzugsweise mehrere Beschleunigungssensoren auf, wobei die Signalverarbeitungseinrichtung mit dem zumindest einen Beschleunigungssensor gekoppelt ist, wobei die Signalverarbeitungseinrichtung dazu eingerichtet ist, Ausgabesignale des zumindest einen Beschleunigungssensors in Tastaturbefehle und/oder in Computermausbefehle umzuwandeln. Vorzugsweise ist der zumindest eine Beschleunigungssensor dazu eingerichtet, Kopfbewegungen eines Verwenders der Eingabeeinrichtung zu erfassen. Diese Ausführungsform der Eingabeeinrichtung ermöglicht es dem Verwender, Tastaturbefehle und/oder Computermausbefehle an den Computer mittels Bewegen der Eingabeeinrichtung, insbesondere mittels Bewegens des mit der Eingabeeinrichtung verbundenen Kopfes, zu übermitteln und auf diese Weise Eingaben zu machen.

Als besonders vorteilhaft wird es angesehen, wenn der zumindest eine Beschleunigungssensor in der Tragevorrichtung gelagert ist. Bevorzugt ist der zumindest eine Beschleunigungssensor ortsveränderlich in der Tragevorrichtung gelagert. Eine derartige Gestaltung ermöglicht es, den Beschleunigungssensor und/oder die Beschleunigungssensoren optimal hinsichtlich der Bewegungsfähigkeit des Verwenders zu positionieren, sodass die Eingabeeinrichtung durch den Verwender besonders komfortabel verwendet werden kann. Bezüglich der Ortsveränderlichkeit des zumindest einen Beschleunigungssensors wird es als besonders vorteilhaft angesehen, wenn dieser verschiebbar in der Tragevorrichtung gelagert ist und/oder die Tragevorrichtung mehrere Haltestrukturen und der Beschleunigungssensor eine zu der jeweiligen Haltestruktur korrespondierende Gegenstruktur aufweist, beispielsweise die jeweilige Haltestruktur und die Gegenstruktur in Art einer Clipsverbindung oder in Art einer Klettverbindung ausgebildet sind. Der Beschleunigungssensor kann somit in einfacher Art und Weise mit derjenigen Haltestruktur der Haltestrukturen verbunden werden, die für die Bewegungsfähigkeit des Verwenders am besten geeignet ist und/oder den persönlichen Vorlieben des Verwenders hinsichtlich der Bedienung der Eingabeeinrichtung entspricht.

In einer vorteilhaften Weiterbildung der Eingabeeinrichtung weist diese zumindest einen Abstandssensor auf, zur Erfassung eines Abstands, wobei die Signalverarbeitungseinrichtung mit dem zumindest einen Abstandssensor gekoppelt ist, wobei die Signalverarbeitungseinrichtung dazu eingerichtet ist, Ausgabesignale des zumindest einen Abstandssensors in Tastaturbefehle und/oder in Computermausbefehle umzuwandeln. Insbesondere dient der Abstandssensor der Erfassung eines Abstands einer Gesichtspartie eines Verwenders von einem Referenzpunkt, wobei der Abstandssensor abhängig vom Abstand der Gesichtspartie des Verwenders vom Referenzpunkt ein Ausgabesignal ausgibt. Beispielsweise kann der Abstandssensor benachbart einer Wange des Verwenders angeordnet sein, sodass der Abstandssensor den Abstand der Wange von einem Referenzpunkt, beispielsweise die Position des Abstandssensors selbst, erfasst, sodass der Verwender durch Aufblasen dieser Wange einen Tastaturbefehl und/oder Mausbefehl an den Computer übermitteln kann.

Als besonders vorteilhaft wird es angesehen, wenn der zumindest eine Abstandssensor, insbesondere ortsveränderlich, in der Tragevorrichtung gelagert ist. Vorzugsweise ist der Abstandssensor mittels eines verstellbaren Arms in der Tragevorrichtung gelagert. Dadurch kann in besonders einfacher Art und Weise der zumindest eine Abstandssensor an die körperlichen Fähigkeiten des Verwenders angepasst werden.

In einer vorteilhaften Ausführungsform weist die Eingabeeinrichtung zumindest einen Näherungssensor auf, wobei die Signalverarbeitungseinrichtung mit dem zumindest einen Näherungssensor gekoppelt ist, wobei die Signalverarbeitungseinrichtung dazu eingerichtet ist, Ausgabesignale des zumindest einen Näherungssensors in Tastaturbefehle und/oder in Computermausbefehle umzuwandeln. Es wird wiederum als vorteilhaft angesehen, wenn der Näherungssensor bei Annäherung einer Gesichtspartie eines Verwenders an den Näherungssensor ein Ausgabesignal ausgibt. Der Näherungssensor ist insbesondere derart ausgebildet, dass dieser bei Annäherung einer Gesichtspartie eines Verwenders ein Ausgabesignal ausgibt.

Der Näherungssensor ist in einer bevorzugten Ausführungsform derart ausgebildet, dass dieser bei Unterschreitung und/oder Überschreitung eines bestimmten Abstandes zum Näherungssensor ein Ausgabesignal ausgibt, wobei dieses Ausgabesignal unabhängig von einem weiteren Annähern bzw. Entfernen ist. Dadurch ist eine besonders einfache Bedienung möglich.

In einer bevorzugten Ausführungsform ist die Signalverarbeitungseinrichtung dazu eingerichtet, bei Gleichzeitigkeit und/oder zeitlicher Überlagerung von Ausgabesignalen und/oder Auftreten von Ausgabesignalen in einem bestimmten Zeitintervall und/oder in einem bestimmten zeitlichem Abstand, die Ausgabesignale in eine Kombination von Tastaturbefehlen und/oder Computermausbefehlen umzuwandeln. Bei dieser Kombination von Tastaturbefehlen und/oder Computermausbefehlen kann es sich beispielsweise um das quasi gleichzeitige Drücken von zwei Tasten einer Tastatur handeln. Es ist aber auch durchaus denkbar, dass die Kombination darin besteht, dass eine Taste der Tastatur quasi dauerhaft gedrückt ist. Dabei ist es durchaus denkbar, dass quasi dauerhafte Drücken der Taste durch entsprechende Ausgabesignale wieder zu beenden oder zeitlich zu befristen.

Die Übertragung der Tastaturbefehle und/oder Computermausbefehle an den Computer erfolgt vorzugsweise kabellos. Insbesondere weist die Signalübertragungseinrichtung ein Funkmodul auf.

Vorzugsweise weist die Eingabeeinrichtung einen Energiespeicher zur Versorgung der Eingabeeinrichtung mit elektrischer Energie auf. Bei dem Energiespeicher kann es sich beispielsweise um einen Akkumulator und/oder eine elektrische Batterie handeln. Der Vorteil bei dieser Ausführungsform der Eingabeeinrichtung besteht darin, dass die Eingabeeinrichtung keine externe Energieversorgung, insbesondere keine externe Stromversorgung mittels eines Kabels, benötigt. Dadurch ist die Verwendung der Eingabeeinrichtung besonders komfortabel möglich.

In einer besonders bevorzugten Ausführungsform der Eingabeeinrichtung weist die Signalverarbeitungseinrichtung einen ersten Betriebsmodus auf und einen zweiten Betriebsmodus auf, wobei in dem ersten Betriebsmodus keine Umwandlung der Ausgabesignale des Mikrofons in Tastaturbefehle und/oder Computermausbefehle erfolgt und/oder keine Übertragung der umgewandelten Tastaturbefehle und/oder Computermausbefehle an den Computer erfolgt und in dem zweiten Betriebsmodus eine Umwandlung der Ausgabesignale des Mikrofons in Tastaturbefehle und/oder in Computermausbefehle erfolgt und eine Übertagung der umgewandelten Tastaturbefehle und/oder Computermausbefehle an den Computer erfolgt. Eine derartige Gestaltung der Eingabeeinrichtung ermöglicht es dem Verwender, das Mikrofon der Eingabeeinrichtung zu verwenden, ohne dass bei der Verwendung des Mikrofons Tastaturbefehle und/oder Computermausbefehle an den Computer übermittelt werden. Dadurch kann der Verwender beispielsweise sprechen, ohne Tastaturbefehle oder Mausbefehle an den Computer zu übermitteln. Dies ist insbesondere dann von Vorteil, wenn der Verwender über den Computer mit anderen Personen spricht, beispielsweise im Rahmen eines Multiplayer-Computerspiels. Das Umschalten zwischen dem ersten Betriebsmodus und dem zweiten Betriebsmodus erfolgt vorzugsweise mittels eines Ausgabesignals oder mittels Ausgabesignalen des Mikrofons und/oder des zumindest einen Beschleunigungssensors und/oder des zumindest einen Abstandssensors und/oder des zumindest einen Näherungssensors. Beispielsweise ist es denkbar, dass durch einen bestimmten Sprachbefehl, z.B. "Betriebsmodus 1", zum ersten Betriebsmodus gewechselt werden kann und mit einem anderem Sprachbefehl, z.B. "Betriebsmodus 2", zum zweiten Betriebsmodus gewechselt werden kann. Ferner ist es denkbar, dass eine bestimmte Kopfbewegung zum Umschalten verwendet wird. Beispielsweise durch Kopfnicken zum ersten Betriebsmodus gewechselt wird und durch Kopfschütteln zum zweiten Betriebsmodus gewechselt wird.

Es ist auch durchaus denkbar, weitere Betriebsmodi vorzusehen, bei denen beispielsweise die Verarbeitung der Ausgabesignale des zumindest einen Beschleunigungssensors und/oder des zumindest einen Abstandssensors und/oder des zumindest einen Näherungssensors deaktiviert ist und in anderen Betriebsmodi die Verarbeitung entsprechend aktiviert ist.

Als besonders vorteilhaft wird es angesehen, wenn die Signalverarbeitungseinrichtung konfigurierbar ist. Unter konfigurierbar wird vorliegend verstanden, dass ein Zusammenhang zwischen Ausgabesignalen des Mikrofons bzw. des Beschleunigungssensors bzw. des Abstandssensors bzw. des Näherungssensors und/oder Kombinationen der Ausgabesignale und den zugeordneten Tastaturbefehlen bzw. Computermausbefehlen veränderbar ist. Es ist auch durchaus denkbar, dass bestimmte Konfigurationen bereits vorhanden bzw. archiviert sind, die dann in die Signalverarbeitungseinrichtung importierbar sind. Ferner ist denkbar, dass die Eingabeeinrichtung dazu eingerichtet ist, Konfigurationen der Signalverarbeitungseinrichtung zu exportieren, insbesondere um diese zu einem späteren Zeitpunkt wieder in die Eingabeeinrichtung zu importieren bzw. auf eine andere Eingabeeinrichtung zu importieren.

In einer vorteilhaften Weiterbildung ist vorgesehen, dass die Eingabeeinrichtung eine Signalempfangseinrichtung aufweist, zum Empfangen von Konfigurationsbefehlen zwecks Konfigurierens der Signalverarbeitungseinrichtung.

Als besonders vorteilhaft wird es angesehen, wenn die Signalverarbeitungseinrichtung mittels eines Computers konfigurierbar ist.

In einer besonders bevorzugten Ausführungsform ist vorgesehen, dass die Eingabeeinrichtung eine Speichereinrichtung aufweist, wobei in der Speichereinrichtung zumindest zwei unterschiedliche Konfigurationen für die Signalverarbeitungseinrichtung gespeichert sind, vorzugsweise die von der Signalverarbeitungseinrichtung zu verwendende Konfiguration durch den Verwender wählbar ist.

Als besonders vorteilhaft wird eine Kombination der Eingabeeinrichtung und einer Computersoftware angesehen, wobei die Computersoftware bei Ausführen der Software auf dem Computer eine Benutzeroberfläche zum Konfigurieren der Eingabeeinrichtung mittels des Computers bereitstellt. Die Software kann beispielsweise zur Administration der Eingabeeinrichtung, zum Speichern von Presets, zum Durchführen von Kalibriervorgängen der Eingabeeinrichtung, zur Durchführung von Software-Updates der Eingabeeinrichtung, zum Definieren von Shortcuts, sowie zum Definieren von Sprachkommandos eingerichtet sein.

Es ist durchaus denkbar, dass die Software dazu eingerichtet ist, bei bestimmten an den Computer übermittelten Tastaturbefehlen und/oder Computermausbefehlen bestimmte Aktionen auszuführen. Bei den Aktionen kann es sich beispielsweise um das Starten oder Beenden eines Programms auf dem Computer handeln.

Die erfindungsgemäße Eingabeeinrichtung ermöglicht es Menschen mit körperlichen Behinderungen, insbesondere Menschen, die an Tetraplegie leiden, unkompliziert einen Computer zu verwenden. Die Eingabeeinrichtung gibt diesen Menschen die Möglichkeit, Computerspiele zu spielen und an der wachsenden e-sport-Szene teilzuhaben. In diesem Zusammenhang wird insbesondere eine Eingabeeinrichtung, die als Headset ausgebildet ist, als vorteilhaft angesehen.

Die Eingabeeinrichtung ist insbesondere derart ausgebildet, dass, bei Herstellen oder nach dem Herstellen einer kabellosen oder kabelgebundenen Kommunikationsverbindung zwischen der Eingabeeinrichtung und dem Computer, die Eingabeeinrichtung von einem auf dem Computer ausgeführten Programm, beispielsweise einem Betriebssystem, als Tastatur und/oder Computermaus erkannt wird. Vorzugsweise werden, bei Herstellen der Kommunikationsverbindung bzw. nach dem Herstellen der Kommunikationsverbindung, auf dem Computer vorinstallierte oder vorhandene Gerätetreiber für eine Tastatur bzw. eine Computermaus verwendet. Es ist somit nicht notwendig, eine spezielle Software zur Verwendung der Eingabeeinrichtung zu installieren. Anstatt dessen werden beispielsweise Gerätetreiber verwendet, die bereits in dem entsprechenden Betriebssystem des Computers hinterlegt sind. Dadurch ist es möglich, die Eingabeeinrichtung universell zu benutzen, da keine Hardwareänderungen, Softwareänderungen bzw. Softwareinstallationen an bzw. auf dem Computer notwendig sind. Dies ist insbesondere dann von Vorteil, wenn aus Sicherheitsgründen eine Installation von Software bzw. Gerätetreibern auf dem zu verwendenden Computer nicht möglich ist oder mit einem erhöhten Aufwand verbunden ist.

In den nachfolgenden Figuren wird die Erfindung anhand von Ausführungsbeispielen näher erläutert, ohne auf diese beschränkt zu sein. Es zeigen:
- Fig. 1: eine Ausführungsform der Eingabeeinrichtung als Headset,
- Fig. 2: eine schematische Darstellung einer weiteren Ausführungsform der Eingabeeinrichtung und eines Computers.

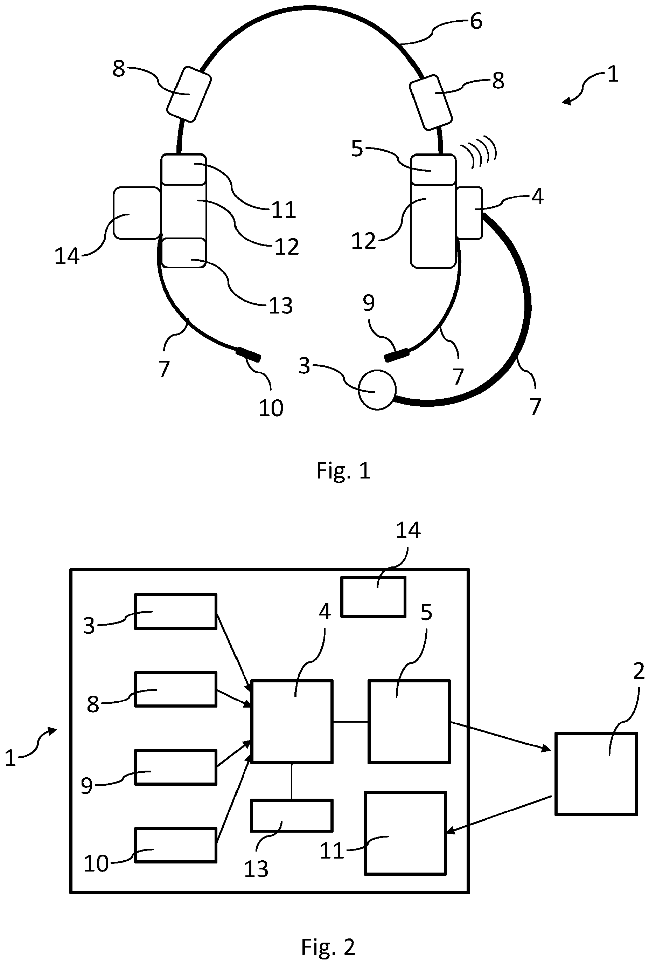

Die Fig. 1 zeigt eine Eingabeeinrichtung 1, die als Headset ausgebildet ist. Die Eingabeeinrichtung 1 dient zur Verwendung mit einem Computer 2. Die Eingabeeinrichtung 1 weist eine Tragevorrichtung 6 in Art eines Bügels auf. Die Eingabeeinrichtung 1 weist zwei Lautsprecher 12 auf, die als Kopfhörer ausgebildet sind, und jeweils an einer Endseite der Tragevorrichtung 6 in der Tragevorrichtung 6 gelagert sind. Die Eingabeeinrichtung 1 umfasst ein Mikrofon 3, das mittels eines verstellbaren Arms 7 in einem der Lautsprecher 12 und somit mittelbar in der Tragevorrichtung 6 gelagert ist. Die Eingabeeinrichtung 1 weist eine Signalverarbeitungseinrichtung 4 auf, wobei die Signalverarbeitungseinrichtung 4 mit dem Mikrofon 3 gekoppelt ist. Die Signalverarbeitungseinrichtung 4 ist dazu eingerichtet, Ausgabesignale des Mikrofons 3 in Tastaturbefehle und/oder in Computermausbefehle umzuwandeln. Die Eingabeeinrichtung 1 weist ferner eine Signalübertragungseinrichtung 5 mit einem Funkmodul auf, zum kabellosen Übertragen der Tastaturbefehle und/oder Computermausbefehle an den Computer 2.

Die Eingabeeinrichtung 1 weist zwei Beschleunigungssensoren 8 auf, wobei die beiden Beschleunigungssensoren 8 verschiebbar in der Tragevorrichtung 6 gelagert sind. Die Signalverarbeitungseinrichtung 4 ist mit den beiden Beschleunigungssensoren 8 gekoppelt, wobei die Signalverarbeitungseinrichtung 4 dazu eingerichtet ist, Ausgabesignale der Beschleunigungssensoren 8 ebenfalls in Tastaturbefehle und/oder in Computermausbefehle umzuwandeln.

Ferner weist die Eingabeeinrichtung 1 einen Abstandssensor 9 und einen Näherungssensor 10 auf, wobei die Abstandssensor 9 und der Näherungssensor 10 jeweils in einem verstellbaren Arm 7 gelagert sind. Die Signalverarbeitungseinrichtung 4 ist mit den beiden Sensoren 9, 10 gekoppelt, wobei die Signalverarbeitungseinrichtung 4 dazu eingerichtet ist, Ausgabesignale des Abstandssensors 9 und Ausgabesignale des Näherungssensors 10 in Tastaturbefehle und/oder in Computermausbefehle umzuwandeln.

Die Eingabeeinrichtung 1 weist darüber hinaus eine Speichereinrichtung 13 auf, wobei in der Speichereinrichtung 13 zumindest zwei unterschiedliche Konfigurationen für die Signalverarbeitungseinrichtung 4 speicherbar sind, wobei zwischen der von der Signalverarbeitungseinrichtung 4 zu verwendenden Konfiguration gewechselt werden kann. Der Wechsel zwischen den Konfigurationen erfolgt vorzugsweise mittels der Ausgabesignale eines oder mehrerer der Komponenten Mikrofon 3, Beschleunigungssensoren 8, Abstandssensor 9 und/oder Näherungssensor 10, die mit der Signalverarbeitungseinrichtung 4 gekoppelt sind.

Die Eingabeeinrichtung 1 umfasst eine Signalempfangseinrichtung 11 zum Empfangen von Konfigurationsbefehlen zwecks Konfigurieren der Signalverarbeitungseinrichtung 4. Ein Konfigurieren der Signalverarbeitungseinrichtung 4 kann beispielsweise mittels des Computers 2 erfolgen.

Die Eingabeeinrichtung 1 weist zudem einen Energiespeicher 14 zur Versorgung der Eingabeeinrichtung 1 mit elektrischer Energie auf.

Der Energiespeicher 14, die Signalempfangseinrichtung 11, die Speichereinrichtung 13, die Signalverarbeitungseinrichtung 4 und die Signalübertragungseinrichtung 5 sind in den Lautsprechern 12 gelagert, vorzugsweise in diese integriert.

Die in der Fig. 1 dargestellte Ausführungsform der Eingabeeinrichtung 1 ermöglicht es dem Verwender, die Eingabeeinrichtung 1 auf dem Kopf zu tragen und den Computer 2 mittels Tastaturbefehlen und/oder Computermausbefehlen zu bedienen, wobei die Eingabeeinrichtung 1 entsprechend der Ausgabesignale des Mikrofons 3 und/oder der Ausgabesignale der Beschleunigungssensoren 8 und/oder der Ausgabesignale des Abstandssensors 9 und/oder der Ausgabesignale des Näherungssensors 10 und entsprechend der Konfiguration der Signalverarbeitungseinrichtung 4 mittels der Signalübertragungseinrichtung 5 Tastaturbefehle und/oder Computermausbefehle an den Computer 2 übermittelt. Auf diese Weise kann der Computer 2 mittels der Eingabeeinrichtung 1 bedient werden, wobei die Eingabeeinrichtung 1 die Tastatur und/oder die Computermaus als Eingabeeinrichtungen ersetzt.

Die Fig. 2 zeigt schematisch eine weitere Ausführungsform der Eingabeeinrichtung 1, wobei diese Eingabeeinrichtung 1 ein Mikrofon 3, einen Beschleunigungssensor 8, einen Abstandssensor 9 und einen Näherungssensor 10 aufweist, die jeweils mit der Signalverarbeitungseinrichtung 4 gekoppelt sind, wobei die Signalverarbeitungseinrichtung 4 dazu eingerichtet ist, die Ausgabesignale der vorgenannten Komponenten 3, 8, 9, 10 in Tastaturbefehle und/oder in Computermausbefehle umzuwandeln. Die Eingabeeinrichtung 1 weist wiederum eine Signalübertragungseinrichtung 5 zum Übertragen der Tastaturbefehle und/oder Computermausbefehle an den Computer 2 auf.

Die Eingabeeinrichtung 1 der Fig. 2 weist darüber hinaus eine Speichereinrichtung 13 auf, wobei in der Speichereinrichtung 13 zumindest zwei unterschiedliche Konfigurationen für die Signalverarbeitungseinrichtung 4 speicherbar sind, wobei zwischen der von der Signalverarbeitungseinrichtung 4 zu verwendenden Konfiguration gewechselt werden kann. Der Wechsel zwischen den Konfigurationen erfolgt vorzugsweise mittels der Ausgabesignale der Komponenten 3, 8, 9, 10, die mit der Signalverarbeitungseinrichtung 4 gekoppelt sind.

Die Eingabeeinrichtung 1 weist zudem einen Energiespeicher 14 zur Versorgung der Eingabeeinrichtung 1 mit elektrischer Energie auf.

Die Eingabeeinrichtung 1 der Fig. 2 umfasst eine Signalempfangseinrichtung 11 zum Empfangen von Konfigurationsbefehlen zwecks Konfigurieren der Signalverarbeitungseinrichtung 4, wobei eine Konfiguration der Signalverarbeitungseinrichtung 4 mittels des Computers 2 erfolgt. Dabei ist es durchaus denkbar, dass zu diesem Zweck auf dem Computer 2 eine speziell auf die Eingabeeinrichtung 1 abgestimmte Software ausgeführt wird.

### Bezugszeichenliste

- 1: Eingabeeinrichtung
- 2: Computer
- 3: Mikrofon
- 4: Signalverarbeitungseinrichtung
- 5: Signalübertragungseinrichtung
- 6: Tragevorrichtung
- 7: Arm
- 8: Beschleunigungssensor
- 9: Abstandssensor
- 10: Näherungssensor
- 11: Signalempfangseinrichtung
- 12: Lautsprecher
- 13: Speichereinrichtung
- 14: Energiespeicher

## Patentansprüche

1. Eingabeeinrichtung (1) zur Verwendung mit einem Computer (2), wobei die Eingabeeinrichtung (1) ein Mikrofon (3) und eine Signalverarbeitungseinrichtung (4) aufweist, wobei die Signalverarbeitungseinrichtung (4) mit dem Mikrofon (3) gekoppelt ist, wobei die Signalverarbeitungseinrichtung (4) dazu eingerichtet ist, Ausgabesignale des Mikrofons (3) in Tastaturbefehle und/oder in Computermausbefehle umzuwandeln, wobei die Eingabeeinrichtung (1) eine Signalübertragungseinrichtung (5) zum Übertragen der Tastaturbefehle und/oder Computermausbefehle an den Computer (2) aufweist.

2. Eingabeeinrichtung nach Anspruch 1, wobei die Eingabeeinrichtung (1) eine Tragevorrichtung (6) aufweist, wobei das Mikrofon (3), vorzugsweise mittels eines verstellbaren Arms (7), in der Tragevorrichtung (6) gelagert ist, wobei die Tragevorrichtung (6) derart ausgebildet ist, dass diese von einem Verwender im Kopfbereich getragen werden kann.

3. Eingabeeinrichtung nach Anspruch 1 oder 2, wobei die Eingabeeinrichtung (1) als Headset ausgebildet ist.

4. Eingabeeinrichtung nach einem der Ansprüche 1 bis 3, wobei die Eingabeeinrichtung (1) zumindest einen Beschleunigungssensor (8), vorzugsweise mehrere Beschleunigungssensoren (8), aufweist, wobei die Signalverarbeitungseinrichtung (4) mit dem zumindest einen Beschleunigungssensor (8) gekoppelt ist, wobei die Signalverarbeitungseinrichtung (4) dazu eingerichtet ist, Ausgabesignale des zumindest einen Beschleunigungssensors (8) in Tastaturbefehle und/oder in Computermausbefehle umzuwandeln.

5. Eingabeeinrichtung nach Anspruch 4, wobei der zumindest eine Beschleunigungssensor (8) in der Tragevorrichtung (6) gelagert ist, vorzugsweise ortsveränderlich in der Tragevorrichtung (6) gelagert ist.

6. Eingabeeinrichtung nach einem der Ansprüche 1 bis 5, wobei die Eingabeeinrichtung (1) zumindest einen Abstandssensor (9) aufweist, zur Erfassung eines Abstands von einem Referenzpunkt, wobei die Signalverarbeitungseinrichtung (4) mit dem zumindest einen Abstandssensor (9) gekoppelt ist, wobei die Signalverarbeitungseinrichtung (4) dazu eingerichtet ist, Ausgabesignale des zumindest einen Abstandssensors (9) in Tastaturbefehle und/oder in Computermausbefehle umzuwandeln.

7. Eingabeeinrichtung nach einem der Ansprüche 1 bis 6, wobei die Eingabeeinrichtung zumindest einen Näherungssensor (10) aufweist, wobei die Signalverarbeitungseinrichtung (4) mit dem zumindest einen Näherungssensor (10) gekoppelt ist, wobei die Signalverarbeitungseinrichtung (4) dazu eingerichtet ist, Ausgabesignale des zumindest einen Näherungssensors (10) in Tastaturbefehle und/oder in Computermausbefehle umzuwandeln.

8. Eingabeeinrichtung nach Anspruch 6 oder 7, wobei der zumindest eine Abstandssensor (9) und/oder der zumindest eine Näherungssensor (10) in der Tragevorrichtung (6) gelagert ist, vorzugsweise mittels eines verstellbaren Arms in der Tragevorrichtung (6) gelagert ist.

9. Eingabeeinrichtung nach einem der Ansprüche 1 bis 8, wobei die Signalverarbeitungseinrichtung (4) dazu eingerichtet ist, bei Gleichzeitigkeit und/oder zeitlicher Überlagerung von Ausgabesignalen und/oder Auftreten von Ausgabesignalen in einem bestimmten Zeitintervall und/oder in einem bestimmten zeitlichem Abstand, die Ausgabesignale in eine Kombination von Tastaturbefehlen und/oder Computermausbefehlen umzuwandeln.

10. Eingabeeinrichtung nach einem der Ansprüche 1 bis 9, wobei die Übertragung der Tastaturbefehle und/oder Computermausbefehle an den Computer (2) kabellos erfolgt, insbesondere die Signalübertragungseinrichtung (5) ein Funkmodul aufweist.

11. Eingabeeinrichtung nach einem der Ansprüche 2 bis 10, wobei die Signalverarbeitungseinrichtung (4) und/oder die Signalübertragungseinrichtung (5) in der Tragevorrichtung (6) gelagert sind.

12. Eingabeeinrichtung nach einem der Ansprüche 1 bis 11, wobei die Signalverarbeitungseinrichtung (4) einen ersten Betriebsmodus aufweist und einen zweiten Betriebsmodus aufweist, wobei in dem ersten Betriebsmodus keine Umwandlung der Ausgabesignale des Mikrofons (3) in Tastaturbefehle und/oder Computermausbefehle erfolgt und/oder keine Übertragung der umgewandelten Tastaturbefehle und/oder Computermausbefehle an den Computer (2) erfolgt und in dem zweiten Betriebsmodus eine Umwandlung der Ausgabesignale des Mikrofons (3) in Tastaturbefehle und/oder Computermausbefehle erfolgt und eine Übertragung der umgewandelten Tastaturbefehle und/oder Computermausbefehle an den Computer (2) erfolgt, vorzugsweise die Signalverarbeitungseinrichtung (4) dazu eingerichtet ist, zwischen dem ersten Betriebsmodus und dem zweiten Betriebsmodus zu wechseln, bei Vorliegen eines bestimmten Ausgabesignals oder bestimmten Ausgabesignalen des Mikrofons (3) und/oder des zumindest einen Beschleunigungssensors (8) und/oder des zumindest einen Abstandssensors (9) und/oder des zumindest einen Näherungssensors (10).

13. Eingabeeinrichtung nach einem der Ansprüche 1 bis 12, wobei die Signalverarbeitungseinrichtung (4) konfigurierbar ist, vorzugsweise die Eingabeeinrichtung (1) eine Signalempfangseinrichtung (11) aufweist, zum Empfangen von Konfigurationsbefehlen zwecks Konfigurierens der Signalverarbeitungseinrichtung (4).

14. Eingabeeinrichtung nach einem der Ansprüche 1 bis 13, wobei die Signalverarbeitungseinrichtung (4) mittels eines Computers (2) konfigurierbar ist.

15. Eingabeeinrichtung nach einem der Ansprüche 1 bis 14, wobei die Eingabeeinrichtung (1) eine Speichereinrichtung (13) aufweist, wobei in der Speichereinrichtung (13) zumindest zwei unterschiedliche Konfigurationen für die Signalverarbeitungseinrichtung (4) gespeichert sind.

## Geänderte Patentansprüche

### Geänderte Patentansprüche gemäss Regel 137(2) EPÜ.

1. Eingabeeinrichtung (1) zur Verwendung mit einem Computer (2), wobei die Eingabeeinrichtung (1) ein Mikrofon (3) und eine Signalverarbeitungseinrichtung (4) aufweist, wobei die Signalverarbeitungseinrichtung (4) mit dem Mikrofon (3) gekoppelt ist, wobei die Signalverarbeitungseinrichtung (4) dazu eingerichtet ist, Ausgabesignale des Mikrofons (3) in Tastaturbefehle und/oder in Computermausbefehle umzuwandeln, wobei die Eingabeeinrichtung (1) eine Signalübertragungseinrichtung (5) zum Übertragen der Tastaturbefehle und/oder Computermausbefehle an den Computer (2) aufweist, **dadurch gekennzeichnet, dass** die Eingabeeinrichtung (1) dazu eingerichtet ist, dass diese von dem verwendeten Computer (2) als Tastatur und/oder Computermaus erkannt wird.

2. Eingabeeinrichtung nach Anspruch 1, wobei die Eingabeeinrichtung (1) eine Tragevorrichtung (6) aufweist, wobei das Mikrofon (3), vorzugsweise mittels eines verstellbaren Arms (7), in der Tragevorrichtung (6) gelagert ist, wobei die Tragevorrichtung (6) derart ausgebildet ist, dass diese von einem Verwender im Kopfbereich getragen werden kann.

3. Eingabeeinrichtung nach Anspruch 1 oder 2, wobei die Eingabeeinrichtung (1) als Headset ausgebildet ist.

4. Eingabeeinrichtung nach einem der Ansprüche 1 bis 3, wobei die Eingabeeinrichtung (1) zumindest einen Beschleunigungssensor (8), vorzugsweise mehrere Beschleunigungssensoren (8), aufweist, wobei die Signalverarbeitungseinrichtung (4) mit dem zumindest einen Beschleunigungssensor (8) gekoppelt ist, wobei die Signalverarbeitungseinrichtung (4) dazu eingerichtet ist, Ausgabesignale des zumindest einen Beschleunigungssensors (8) in Tastaturbefehle und/oder in Computermausbefehle umzuwandeln.

5. Eingabeeinrichtung nach Anspruch 4, wobei der zumindest eine Beschleunigungssensor (8) in der Tragevorrichtung (6) gelagert ist, vorzugsweise ortsveränderlich in der Tragevorrichtung (6) gelagert ist.

6. Eingabeeinrichtung nach einem der Ansprüche 1 bis 5, wobei die Eingabeeinrichtung (1) zumindest einen Abstandssensor (9) aufweist, zur Erfassung eines Abstands von einem Referenzpunkt, wobei die Signalverarbeitungseinrichtung (4) mit dem zumindest einen Abstandssensor (9) gekoppelt ist, wobei die Signalverarbeitungseinrichtung (4) dazu eingerichtet ist, Ausgabesignale des zumindest einen Abstandssensors (9) in Tastaturbefehle und/oder in Computermausbefehle umzuwandeln.

7. Eingabeeinrichtung nach einem der Ansprüche 1 bis 6, wobei die Eingabeeinrichtung zumindest einen Näherungssensor (10) aufweist, wobei die Signalverarbeitungseinrichtung (4) mit dem zumindest einen Näherungssensor (10) gekoppelt ist, wobei die Signalverarbeitungseinrichtung (4) dazu eingerichtet ist, Ausgabesignale des zumindest einen Näherungssensors (10) in Tastaturbefehle und/oder in Computermausbefehle umzuwandeln.

8. Eingabeeinrichtung nach Anspruch 6 oder 7, wobei der zumindest eine Abstandssensor (9) und/oder der zumindest eine Näherungssensor (10) in der Tragevorrichtung (6) gelagert ist, vorzugsweise mittels eines verstellbaren Arms in der Tragevorrichtung (6) gelagert ist.

9. Eingabeeinrichtung nach einem der Ansprüche 1 bis 8, wobei die Signalverarbeitungseinrichtung (4) dazu eingerichtet ist, bei Gleichzeitigkeit und/oder zeitlicher Überlagerung von Ausgabesignalen und/oder Auftreten von Ausgabesignalen in einem bestimmten Zeitintervall und/oder in einem bestimmten zeitlichem Abstand, die Ausgabesignale in eine Kombination von Tastaturbefehlen und/oder Computermausbefehlen umzuwandeln.

10. Eingabeeinrichtung nach einem der Ansprüche 1 bis 9, wobei die Übertragung der Tastaturbefehle und/oder Computermausbefehle an den Computer (2) kabellos erfolgt, insbesondere die Signalübertragungseinrichtung (5) ein Funkmodul aufweist.

11. Eingabeeinrichtung nach einem der Ansprüche 2 bis 10, wobei die Signalverarbeitungseinrichtung (4) und/oder die Signalübertragungseinrichtung (5) in der Tragevorrichtung (6) gelagert sind.

12. Eingabeeinrichtung nach einem der Ansprüche 1 bis 11, wobei die Signalverarbeitungseinrichtung (4) einen ersten Betriebsmodus aufweist und einen zweiten Betriebsmodus aufweist, wobei in dem ersten Betriebsmodus keine Umwandlung der Ausgabesignale des Mikrofons (3) in Tastaturbefehle und/oder Computermausbefehle erfolgt und/oder keine Übertragung der umgewandelten Tastaturbefehle und/oder Computermausbefehle an den Computer (2) erfolgt und in dem zweiten Betriebsmodus eine Umwandlung der Ausgabesignale des Mikrofons (3) in Tastaturbefehle und/oder Computermausbefehle erfolgt und eine Übertragung der umgewandelten Tastaturbefehle und/oder Computermausbefehle an den Computer (2) erfolgt, vorzugsweise die Signalverarbeitungseinrichtung (4) dazu eingerichtet ist, zwischen dem ersten Betriebsmodus und dem zweiten Betriebsmodus zu wechseln, bei Vorliegen eines bestimmten Ausgabesignals oder bestimmten Ausgabesignalen des Mikrofons (3) und/oder des zumindest einen Beschleunigungssensors (8) und/oder des zumindest einen Abstandssensors (9) und/oder des zumindest einen Näherungssensors (10).

13. Eingabeeinrichtung nach einem der Ansprüche 1 bis 12, wobei die Signalverarbeitungseinrichtung (4) konfigurierbar ist, vorzugsweise die Eingabeeinrichtung (1) eine Signalempfangseinrichtung (11) aufweist, zum Empfangen von Konfigurationsbefehlen zwecks Konfigurierens der Signalverarbeitungseinrichtung (4).

14. Eingabeeinrichtung nach einem der Ansprüche 1 bis 13, wobei die Signalverarbeitungseinrichtung (4) mittels eines Computers (2) konfigurierbar ist.

15. Eingabeeinrichtung nach einem der Ansprüche 1 bis 14, wobei die Eingabeeinrichtung (1) eine Speichereinrichtung (13) aufweist, wobei in der Speichereinrichtung (13) zumindest zwei unterschiedliche Konfigurationen für die Signalverarbeitungseinrichtung (4) gespeichert sind.
